# EUROPEAN PATENT APPLICATION

(11) **EP 1 160 254 A1**
(43) Date of publication of application: **05.12.2001**
(21) Application number: 01304663.6
(22) Date of filing: 25.05.2001
(51) Int. Cl.: C07K 14/435, C07K 14/705, C07K 16/28, C12N 15/11, C12Q 1/68, C12N 15/66, A61K 38/17, A61P 23/00

(54) **Human vanilloid receptor-like proteins**

(30) Priority: 31.05.2000 US 208156 P
(71) Applicant: PFIZER INC., New York, N.Y. 10017 (US)
(72) Inventor: Shinjo, Katsuhiro, Pfizer Global Res. and Develop., Chita-gun, Aichi-ken 470-2393 (JP); Yabuuchi, Hikaru, Sagamihara-shi, Kanagawa 228-0811 (JP)
(74) Representative: Hayles, James Richard

(57) **Abstract**

This invention relates to human vanilloid receptor-like protein 2 (VRL-2) polypeptides, polynucleotides encoding such polypeptides, polynucleotide probes or primers, expression vectors and host cells comprising such DNA molecules. This invention further relates to a process for producing the polypeptides; an antibody immunospecific for the polypeptide; a diagnostic kit for diagnosing the VRL-2 receptor related disease; a method for screening to identify modulators which modulate the polypeptides; modulators identified by the screening method; a pharmaceutical composition for treatment of conditions associated with biological function of the polypeptides; and a non-human transgenic animal model for vanilloid receptor-like gene. The polypeptides and the DNA molecules of the present invention can be used to identify agonists, antagonists or the like. These agonists and antagonists are useful for treatment of diseases such as pain, nociceptive pain, chronic pain, neuropathic pain, postoperative pain, cancer pain, rheumatoid arthritic pain, osteoarthritis, diabetic neuropathies, neuralgia, neuropathies, algesia, nerve injury, muscle-skeletal pain, low back pain, neurodegeneration, stroke, inflammatory disorders, asthma, allergy, urogenital disorders, incontinence, hypertension, hypotension, perivasular disease and the like.

## Description

### Technical Field

This invention relates to human vanilloid receptor-like protein 2 (VRL-2) polypeptides, polynucleotides encoding such polypeptides, polynucleotide probes or primers, expression vectors and host cells comprising such DNA molecules. This invention further relates to a process for producing the polypeptides; an antibody immunospecific for the polypeptide; a diagnostic kit for diagnosing the VRL-2 receptor related disease; a method for screening to identify modulators which modulate the polypeptides; modulators obtainable by the screening method; a pharmaceutical composition for treatment of conditions associated with biological function of the polypeptides; and a non-human transgenic animal model for vanilloid receptor-like gene.

### Background Art

The analgesic properties of capsaicin and capsaicinoides are known for their uses in the treatment of a variety of disorders such as pain, chronic pain, neuropathic pain, postoperative pain, rheumatoid arthritic pain, neuralgia, neuropathies, algesia, nerve injury, ischaemia, neurodegeneration stroke incontinence and inflammatory disorders (e.g., Campbell et al. "Clinical Applications of Capsaicin and Its Analogues" in Capsaicin in the Study of Pain, Academic Press pgs. 255-272 (1993)). Capsaicin receptors are believed to be members of the ion channel family of polypeptides. These receptors are believed to be associated with the mechanism of action of capsaicin (a vanilloid compound). Capsaicin elicits a senstation of burning pain by selectively activating sensory neurons that convey information about noxious stimuli to the central nervous system (e.g., Caterina, M. J. eta al., "The Capsaicin Receptor: A Heat Activated Ion Channel In the Pain Pathway", Nature 389, 816-824 (1997) and Caterina, M. J. et al., "A Capsaicin-Receptor Homologue with A High Threshold For Noxious Heat", Nature 398, 436-441 (1999)). The channels are permeable to cations and exhibit a notable preferance for divalent cations, particularly calcium ions. The level of calcium ion permeability exceeds that observed for most non-selective cation channels and is similar to values observed for NMDA-type glutamate receptors and alpha-7 nicotinic acetylcholine receptors.

Some polynucleotides encoding vanilloid receptors or vanilloid-like receptors have been cloned and identified (Caterina, M. J. eta al., Nature 389, 816-824 (1997) and Caterina, M. J. et al., Nature 398, 436-441 (1999)). PCT Application International Publication No. WO 99/37675 discloses vanilloid receptor polypeptides and vanilloid receptor-related polypeptides, specifically the capsaicin receptor subtypes VR1 and VR2, and the encoding polynucleotide sequences. PCT Application International Publication NO. WO 99/37765 discloses VANILREP2 polypeptides and polynucleotides, recombinant materials and methods for their production.

If other subtypes of the capsaicin receptors were found and identified, it would be beneficial, for example, to use such subtypes to find novel ligands with respect to the capsaicin receptors.

### Brief Disclosure of the Invention

The present invention provides a polypeptide selected from:
(a) a polypeptide comprising an amino acid sequence of SEQ ID NO:2 or SEQ ID NO:4, or variants thereof; and
(b) a polypeptide having the deduced amino acid sequence translated from the polynucleotide sequence SEQ ID NO:1 or SEQ ID NO:3, or variants thereof.

Such polypeptides have been identified as novel human vanilloid receptor-like protein 2 polypeptides. As used herein, the novel human vanilloid receptor-like protein 2 is referred to as "VRL-2" or "VRL-2 protein". As used herein, these polypetides can be referred to as "VRL-2 polypeptides" or "VRL-2 receptor polypeptides".

The present invention also provides an isolated and/or purified polynucleotide selected from:
(a) a polynucleotide encoding the polypeptide comprising an amino acid sequence of SEQ ID NO:2 or SEQ ID NO:4;
(b) a polynucleotide having a nucleotide sequence of the 85th to 2700th nucleotides in the nucleotide sequence of SEQ ID NO:1, or a nucleotide sequence of the 43rd to 1851st nucleotides in the nucleotide sequence of SEQ ID NO:3 or variants thereof;
(c) a polynucleotide comprising a nucleotide sequence that has at least 70% identity to the polynucleotide of (a) or (b);
(d) a complement to the polynucleotide of any one of (a) to (c);
(e) a polynucleotide comprising a nucleotide sequence which is capable of hybridising to the polynucleotide of any one of (a) to (d); and
(f) a polynucleotide fragment of the polynucleotide of any one of (a) to (e).

The present invention also provides a polynucleotide probe or primer comprising at least 15 contiguous nucleotides of the polynucleotide described above, or the complement thereof.

Further, the present invention provides an expression vector for the expression of the VRL-2 protein in a recombinant host cell wherein said expression vector contains the above DNA molecule, and a host cell which expresses a recombinant VRL-2 protein wherein said host cell contains the expression vector. Also provided is a process for producing the VRL-2 receptor polypeptides, which comprises culturing the host cell under conditions sufficient for the production of said polypeptide and recovering the polypeptide from the culture medium.

The VRL-2 receptor polypeptides, the VRL-2 receptor-encoding DNA, the expression vectors and recombinant host cells expressing recombinant VRL-2 are useful in the identification of modulators which modulate the function of the VRL-2 receptor. Thus, the present invention also provides a method for screening to identify modulators which modulate (e.g., bind) the VRL-2 receptor polypeptide, which comprises contacting the polypeptide with a test compound or sample, and detecting an activity of the compound or sample to modulate the function of the VRL-2 receptor polypeptide.

The present invention further provides a VRL-2 receptor modulator (e.g., agonist and antagonist) obtainable by the above screening method. These VRL-2 receptor modulators are useful for the treatment of diseases or conditions associated with the function of the VRL-2 receptor, or inappropriate VRL-2 receptor activity. Such diseases include pain, nociceptive pain, chronic pain, neuropathic pain, postoperative pain, cancer pain, rheumatoid arthritic pain, osteoarthritis, diabetic neuropathies, neuralgia, neuropathies, algesia, nerve injury, muscle-skeletal pain, low back pain, neurodegeneration, stroke, inflammatory disorders, asthma, allergy, urogenital disorders, incontinence, hypertension, hypotension, perivasular disease and the like.

Thus, the VRL-2 receptor modulators are useful in the treatment of such diseases. In addition, the VRL-2 receptor polypeptides and the VRL-2 receptor-encoding polynucleotides are also useful in diagnostic assays for detecting the VRL-2 receptor-related diseases.

The present invention further provides a diagnostic kit for diagnosing a disease or a susceptibility to a disease associated with biological function of a human vanilloid receptor-like protein, which comprises the above polynucleotides or an antibody to the VRL-2 receptor polypeptide.

In addition, the present invention provides an antibody immunospecific for the VRL-2 receptor polypeptides, and a non-human transgenic animal model for VRL-2 receptor gene function wherein the transgenic animal has an alteration in VRL-2 receptor function relative to a normal animal of the same species.

### Brief Description of Figures

Figs.1(A) and 1(B) show Ca²⁺ increase in hVRL-2 transfected CHO-K1 cells in response to a reduction in extracellular osmolarity.

Figs.2(A) and 2(B) show Ca²⁺ increase in hVRL-2 transfected HEK293 cells in response to a reduction in extracellular osmolarity.

### Detailed Description of the Invention

As used herein, the term "nucleotide sequence" refers to an oligonucleotide sequence or polynucleotide sequence, and variants, homologues, fragments and derivatives thereof (such as portions thereof). The nucleotide sequence may be DNA or RNA of genomic or synthetic or recombinant origin, which may be doublestranded or single-stranded whether representing the sense or antisense strand. Preferably, the term "nucleotide sequence" means DNA. More preferably, the term "nucleotide sequence" means DNA prepared by use of recombinant DNA techniques (i.e. recombinant DNA).

As used herein "amino acid sequence" refers to peptide or protein sequences or portions thereof.

As used herein, the terms "isolated" and "purified" refer to molecules, either nucleic or amino acid sequences, that are removed from their natural environment and isolated or separated from at least one other component with which they are naturally associated.

The terms "variant" in relation to the amino acid sequence for the preferred polypeptide of the present invention include any substitution of, variation of, modification of, replacement of, deletion of or addition of one or more amino acids from or to the sequence providing the resultant polypeptide has VRL-2 activity.

The terms "variant" or "fragment" in relation to the nucleotide sequence coding for the preferred polypeptide of the present invention include any substitution of, variation of, modification of, replacement of, deletion of or addition of one or more nucleic acids from or to the sequence providing the resultant nucleotide sequence codes for or is capable of coding for a polypeptide having VRL-2 activity. Relative sequence homology (i.e. sequence identity) can be determined by commercially available computer programs that can calculate % homology between two or more sequences. A typical example of such a computer program is CLUSTAL.

The term "variant" encompasses sequences that are complementary to sequences that are capable of hybridising under stringent conditions (e.g. 65°C and 0.1xSSC {1xSSC = 0.15 M NaCI, 0.015 M Na₃ citrate pH 7.0}) to the nucleotide sequences presented herein.

The term "expression vector" means a construct capable of *in vivo* or *in vitro* expression.

As used herein, the term "antibodies" include polyclonal and monoclonal antibodies, chimeric, single chain and humanized antibodies. The term "immunospecific" means that the antibodies have substantially greater affinity for the polypeptides of the invention than their affinity for other related polypeptides.

The present invention will be further described bellow.

The present invention is based on the identification of a polynucleotide sequence encoding a novel human vanilloid receptor-like protein 2 (VRL-2) polypeptide. Such a polynucleotide has been isolated and purified, and the nucleotide sequence has been deteremined.

### VRL-2 Receptor Polypeptides

In a first aspect, the present invention provides a polypeptide selected from:
(a) a polypeptide comprising an amino acid sequence of SEQ ID NO:2 or SEQ ID NO:4, or variants thereof; and
(b) a polypeptide having the deduced amino acid sequence translated from the polynucleotide sequence SEQ ID NO:1 or SEQ ID NO:3, or variants thereof.

The VRL-2 receptor polypeptides of the present invention are believed to be members of the ion channel family of polypeptides. The channels are permeable to cations and exhibit a notable preferance for divalent cations, particularly calcium ions. The permeability of cations and the signal transmission ability of the VRL-2 receptors are affected by a ligand which modulates (e.g., binds, inhibits and activates) the function of the VRL-2 receptor polypeptides. These properties are hereinafter referred to as "VRL-2 receptor activities".

Preferably, the polypeptide or variants comprises an amino acid sequence that has at least 75% (more preferably at least 85%, even more preferably at least 95%) identity to the an amino acid sequence of SEQ ID NO:2 or SEQ ID NO:4. The variants preferably show at least one of the above-mentioned VRL-2 activities. More specifically, the polypeptides may vary from the referents by conservative amino acid substitutions, whereby a residue is substituted by another with the VRL-2 activity. Such substitutions may be made among Ala, Val, Leu and lle; among Ser and Thr; among the acidic residues Asp and Glu; among Asn and Gln; and among the basic residues Lys and Arg; or aromatic residues Phe and Tyr. Thus, this invention includes variants of the polypeptides where 1 to 10, more preferably 1 to 5, further preferably 1 to 3, further preferably 1 to 2 or most preferably 1 amino acid(s) are substituted, deleted, or added in any combination in the amino acid sequence of SEQ ID NO:2 or NO:4.

The polypeptides of the present invention may be in the form of the "mature" protein or may be a part of a larger protein such as a precursor or a fusion protein. It is often advantageous to include an additional amino acid sequence which contains secretory or leader sequences, prosequences, sequences which aid in purification such as multiple histidine residues, or an additional sequence for stability during recombinant production.

Polypeptides of the present invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.□□The VRL-2 receptor polypeptides of this invention can be prepared using DNA molecules encoding the amino acid sequence of the polypeptides.

### Polynucleotides/DNA molecules

In a further aspect, the present invention relates to an isolated and/or purified polynucleotide selected from:
(a) a polynucleotide encoding the polypeptide comprising an amino acid sequence of SEQ ID NO:2 or SEQ ID NO:4;
(b) a polynucleotide having a nucleotide sequence of the 85th to 2700th nucleotides in the nucleotide sequence of SEQ ID NO:1, or a nucleotide sequence of the 43rd to 1851st nucleotides in the nucleotide sequence of SEQ ID NO:3 or variants thereof;
(c) a polynucleotide comprising a nucleotide sequence that has at least 70% identity to the polynucleotide of (a) or (b);
(d) a complement to the polynucleotide of any one of (a) to (c);
(e) a polynucleotide comprising a nucleotide sequence which is capable of hybridising to the polynucleotide of any one of (a) to (d); and
(f) a polynucleotide fragment of the polynucleotide of any one of (a) to (e).

Preferably, the polynucleotide comprises a nucleotide sequence that has at least 75% (more preferably at least 85%, yet more preferably at least 95%) identity to the nucleotide sequence of the above polynucleotide (b).

The nucleotide sequences of SEQ ID NO: 1 and SEQ ID NO:3 show homology with rat vanilloid receptor 1 (VR1) (M. J. Caterina et al., Nature 389: 816-824, 1997), rat/human vanilloid receptor like protein 1 (VRL-1) (M. J. Caterina et al., Nature 398: 436-441, 1999), rat stretch-activated channel (SIC) (J. Biol. Chem. 274: 6330-6335, 1999) and mouse growth-factor-regulated channel (GRC) (Nature Cell Biol. 1: 165-170). The nucleotide sequences of SEQ ID NO:1 and SEQ ID NO:3 were found to be full length of cDNA encoding the VRL-2a and VRL-2b polypeptides, respectively. Similarities of human VRL-2a with rat VR1, rat VRL-1, human VRL-1, rat SIC and mouse GRC in amino acid sequence were 51%, 46%, 47%, 59% and 47%, respectively. Similarities of human VRL-2b with rat VR1, rat VRL-1, human VRL-1, rat SIC and mouse GRC in amino acid sequence were 50%, 45%, 46%, 59% and 45%, respectively. VR1 is heat, proton and capsaicin-activated cation channel. Rat VRL-1 is heat-activated cation channel. Rat SIC is mechano-sensitive cation channel. Mouse GRC is IGF-1-regulated cation channel. Accordingly, both human VRL-2a and VRL-2b are predicted to be cation channels activated by stumuli such as heat, proton, chemicals, mechano-stimulation of membrane, growth factor.

The polynucleotides (DNA molecules) of the present invention may be obtained, using standard cloning and screening techniques, from a cDNA library derived from mRNA in cells of human placenta, brain, heart, melanocytse, liver, spleen, foreskin, lung, parathyroid, tonsil, whole embryo, Jurkat T-cells, B-cells and testes (for example Sambrook et al., Molecular Cloning: A Laboratory Manual, 2nd Ed., Cold Spring Harbor Laboratory Press, Cold Spring Harbor, N.Y.(1 989)). The DNA molecules of the invention can also be obtained from natural sources such as genomic DNA libraries or can be synthesized using well known and commercially available techniques.

Other cells and cell lines may also be suitable for use to isolate VRL-2 cDNA. Selection of suitable cells may be done by screening for VRL-2 on cell surfaces. Methods for detecting VRL-2 activity are well known in the art and measure the binding of radiolabelled ligand specific for the receptor. Cells which possess VRL-2 activity in this assay may be suitable for the isolation of VRL-2 cDNA.

Any of a variety of procedures may be used to clone VRL-2 cDNA. These methods include, but are not limited to, direct functional expression of the VRL-2 cDNA following the construction of an VRL-2-containing cDNA library in an appropriate expression vector system. Another method is to screen an VRL-2-containing cDNA library constructed in a bacteriophage or plasmid shuttle vector with a labelled oligonucleotide probe designed from the amino acid sequence of the VRL-2 protein. The preferred method includes screening an VRL-2-containing cDNA library constructed in a bacteriophage or plasmid shuttle vector with a partial cDNA encoding the VRL-2 protein. This partial cDNA is obtained by the specific PCR amplification of VRL-2 DNA fragments through the design of degenerate oligonucleotide primers from the amino acid sequence known for other capsaicin receptors.

It is readily apparent to those skilled in the art that other types of libraries, as well as libraries constructed from other cells or cell types, may be useful for isolating VRL-2-encoding DNA. Other types of libraries include, but are not limited to, cDNA libraries derived from other cells or cell lines other than human erythroleukemia cells, and genomic DNA libraries.

It is readily apparent to those skilled in the art that suitable cDNA libraries may be prepared from cells or cell lines which have VRL-2 activity. The selection of cells or cell lines for use in preparing a cDNA library to isolate VRL-2 cDNA may be done by first measuring cell associated VRL-2 activity using the known labelled ligand binding assay cited above and used herein.

Preparation of cDNA libraries can be performed by standard techniques well known in the art. Well known cDNA library construction techniques can be found for example, in Maniatis, T., Fritsch, E. F., Sambrook, J., Molecular Cloning: A Laboratory Manual (Cold Spring Harbor Laboratory, Cold Spring Harbor, N.Y., 1982).

It is also readily apparent to those skilled in the art that DNA encoding VRL-2 may also be isolated from a suitable genomic DNA library. Construction of genomic DNA libraries can be performed by standard techniques well known in the art. Well known genomic DNA library construction techniques can be found in Maniatis, T., Fritsch, E. F., Sambrook, J. in Molecular Cloning: A Laboratory Manual (Cold Spring Harbor Laboratory, Cold Spring Harbor, N.Y., 1982).

In order to clone the VRL-2 gene by one of the preferred methods, the amino acid sequence or DNA sequence of VRL-2 or a homologous protein is necessary. To accomplish this, VRL-2 protein or a homologous protein may be purified and partial amino acid sequence determined by automated sequenators. It is not necessary to determine the entire amino acid sequence, but the linear sequence of two regions of 6 to 8 amino acids can be determined for the PCR amplification of a partial VRL-2 DNA fragment.

Once suitable amino acid sequences have been identified, the DNA sequences capable of encoding them are synthesized. Because the genetic code is degenerate, more than one codon may be used to encode a particular amino acid, and therefore, the amino acid sequence can be encoded by any of a set of similar DNA oligonucleotides. Only one member of the set will be identical to the VRL-2 sequence but others in the set will be capable of hybridizing to VRL-2 DNA even in the presence of DNA oligonucleotides with mismatches. The mismatched DNA oligonucleotides may still sufficiently hybridize to the VRL-2 DNA to permit identification and isolation of VRL-2 encoding DNA.

Using one of the preferred methods, cDNA clones encoding VRL-2 polypeptide are isolated in a two-stage approach employing polymerase chain reaction (PCR) based technology and cDNA library screening. In the first stage, NH.sub.2-terminal and internal amino acid sequence information from the purified VRL-2 or a homologous protein is used to design degenerate oligonucleotide primers for the amplification of VRL-2-specific DNA fragments. In the second stage, these fragments are cloned to serve as probes for the isolation of full length cDNA from a cDNA library derived from human erythroleukemia cells.

The polynucleotides of the present invention may be used as hybridization probes for cDNA and genomic DNA or as primers for a nucleic acid amplification (PCR) reaction, to isolate full-length cDNAs and genomic clones encoding polypeptides of the present invention and to isolate cDNA and genomic clones of other genes (including genes encoding paralogs from human sources and orthologs and paralogs from species other than human) that have a high sequence similarity to SEQ ID NO:1 or SEQ ID NO:3.

The probes or primers of the present invention generally comprise at least 15 nucleotides, preferably, at least 30 nucleotides and may have at least 50 nucleotides. Particularly preferred probes will have between 30 and 50 nucleotides. Particularly preferred primers will have between 20 and 25 nucleotides.

A polynucleotide encoding a polypeptide of the present invention, including homologs from species other than human, may be obtained by a process which comprises the steps of screening an appropriate library under stringent hybridization conditions with a labeled probe having the sequence of SEQ ID NO:1 or SEQ ID NO:3 or a fragment thereof and isolating full-length cDNA and genomic clones containing said polynucleotide sequence. Such hybridization techniques are well known to the skilled artisan. Preferred stringent hybridization conditions include overnight incubation at 42°C in a solution comprising: 50% formamide, 5xSSC (150mM NaCl, 15mM trisodium citrate), 50 mM sodium phosphate (pH7.6), 5x Denhardt's solution, 10% dextran sulfate, and 20 microgram/ml denatured, sheared salmon sperm DNA; followed by washing the filters in 0.1 x SSC at about 65°C. Thus the present invention also includes polynucleotides obtainable by screening an appropriate library under stringent hybridization conditions with a labeled probe having the sequence of SEQ ID NO:1 or SEQ ID NO:3, or a fragment thereof.

The skilled artisan will appreciate that, in many cases, an isolated cDNA sequence will be incomplete, in that the region coding for the polypeptide is short at the 5' end of the cDNA. This is a consequence of reverse transcriptase, an enzyme with inherently low 'processivity' (a measure of the ability of the enzyme to remain attached to the template during the polymerization reaction), failing to complete a DNA copy of the mRNA template during 1st strand cDNA synthesis.

There are several methods available and well known to those skilled in the art to obtain full-length cDNAs, or extend short cDNAs, for example those based on the method of Rapid Amplification of cDNA ends (RACE) (see, for example. Frohman et al., PNAS USA 85, 8998-9002, 1988). Recent modifications of the technique, exemplified by the Marathon™ technology (Clontech Laboratories Inc.) for example, have significantly simplified the search for longer cDNAs.

### Expression Vectors

Recombinant VRL-2 polypeptides of the present invention may be prepared by processes well known in the art from genetically engineered host cells comprising expression vectors. Accordingly, the present invention relates to an expression vector for the expression of a human vanilloid receptor-ike protein in a recombinant host cell wherein said expression vector contains the above-identified DNA molecule.

The cloned VRL-2 cDNA obtained through the methods described above may be recombinantly expressed by molecular cloning into an expression vector containing a suitable promoter and other appropriate transcription regulatory elements, and transferred into prokaryotic or eukaryotic host cells to produce recombinant VRL-2 polypeptides. Techniques for such manipulations can be found described in Maniatis, T, et al., supra, and are well known in the art.

Expression vectors are defined herein as DNA sequences that are required for the transcription of cloned DNA and the translation of their mRNAs in an appropriate host. Such vectors can be used to express eukaryotic DNA in a variety of hosts such as bacteria, bluegreen algae, plant cells, insect cells and animal cells.

Specifically designed vectors allow the shuttling of DNA between hosts such as bacteria-yeast or bacteria-animal cells. An appropriately constructed expression vector should contain an origin of replication for autonomous replication in host cells, selectable markers, a limited number of useful restriction enzyme sites, a potential for high copy number, and active promoters. A promoter is defined as a DNA sequence that directs RNA polymerase to bind to DNA and initiate RNA synthesis. A strong promoter is one which causes mRNAs to be initiated at high frequency. Expression vectors may include, but are not limited to, cloning vectors, modified cloning vectors, specifically designed plasmids or viruses.

A variety of mammalian expression vectors may be used to express recombinant VRL-2 in mammalian cells. Commercially available mammalian expression vectors which may be suitable for recombinant VRL-2 expression, include but are not limited to, pMClneo (Stratagene), pXT1 (Stratagene), pSG5 (Stratagene), pcDNA1, pcDNA3.1 (Invitrogen), EBO-pSV2-neo (ATCC 37593) pBPV-1(8-2) (ATCC 37110), pdBPV-MMTneo(342-12) (ATCC 37224), pRSVgpt (ATCC 37199), pRSVneo (ATCC 37198), pSV2-dhfr (ATCC 37146), pUCTag (ATCC 37460), and IZD35 (ATCC 37565).

### Host Cells Containing Expression Vector

DNA encoding the VRL-2 polypeptide may also be cloned into an expression vector for expression in a host cell. Host cells may be prokaryotic or eukaryotic, including but not limited to bacteria, fungal, insect, animal (including mammalian) and plant cells. Suitable bacterial cells inlcude *Streptococci, Staphylococci*, *E. coli, Streptomyces* and *Bacillus subtilis* cells. Suitable fungal cells include yeast cells and *Aspergillus* cells. The animal cells include cell lines of human, bovine, porcine, monkey and rodent origin. Suitable insect cells include *Drosophila* S2 and *Spodoptera* Sf9 cells. Suitable animal cells include CHO, COS, HeLa, C127, 3T3, BHK, HEK 293 and Bowes melanoma cells. Cell lines derived from mammalian species which may be suitable and which are commercially available, include CV-1 (ATCC CCL 70), COS-1 (ATCC CRL 1650), COS-7 (ATCC CRL 1651), CHO-K1 (ATCC CCL 61), 3T3 (ATCC CCL 92), NIH/3T3 (ATCC CRL 1658), HeLa (ATCC CCL 2), C1271 (ATCC CRL 1616), BS-C-1 (ATCC CCL 26) and MRC-5 (ATCC CCL 171).

The expression vector may be introduced into host cells via any one of a number of techniques including but not limited to transformation, transfection, protoplast fusion, and electroporation. The expression vector-containing cells are individually analyzed to determine whether they produce VRL-2 protein. Identification of VRL-2 expressing cells may be done by several means, including but not limited to immunological reactivity with anti-VRL-2 antibodies, and the presence of host cell-associated VRL-2 activity.

Expression of VRL-2 DNA may also be performed using in vitro produced synthetic mRNA. Synthetic mRNA can be efficiently translated in various cell-free systems, including but not limited to wheat germ extracts and reticulocyte extracts, as well as efficiently translated in cell based systems, including but not limited to microinjection into frog oocytes, with microinjection into frog oocytes being preferred.

To determine the VRL-2 cDNA sequence(s) that yields optimal levels of receptor activity and/or VRL-2, VRL-2 cDNA molecules including but not limited to the following can be constructed: the full-length open reading frame of the VRL-2 cDNA and various constructs containing portions of the cDNA encoding only specific domains of the receptor protein or rearranged domains of the protein. All constructs can be designed to contain none, all or portions of the 5' and/or 3' untranslated region of VRL-2 cDNA. VRL-2 activity and levels of protein expression can be determined following the introduction, both singly and in combination, of these constructs into appropriate host cells. Following determination of the VRL-2 cDNA cassette yielding optimal expression in transient assays, this VRL-2 cDNA construct is transferred to a variety of expression vectors (including recombinant viruses), including but not limited to those for mammalian cells, plant cells, insect cells, oocytes, *E. Coli,* and yeast cells.

Mammalian cell transfectants are assayed for both the levels of VRL-2 receptor activity and levels of VRL-2 protein by the following methods. Assessing VRL-2 receptor activity involves the direct introduction of a labelled ligand to the cells and determining the amount of specific binding of the ligand to the VRL-2-expressing cells. Binding assays for receptor activity are known in the art (Frey et al., 1993, Eur. J. Pharmacol., 244, pp 239-250).

Levels of VRL-2 protein in host cells is quantitated by a variety of techniques including, but not limited to, immunoaffinity and/or ligand affinity techniques. VRL-2-specific affinity beads or VRL-2-specific antibodies are used to isolate .sup.35 S-methionine labelled or unlabelled VRL-2 protein. Labelled VRL-2 protein is analyzed by SDS-PAGE. Unlabelled VRL-2 protein is detected by Western blotting, ELISA or RIA assays employing VRL-2 specific antibodies.

Following expression of VRL-2 in a host cell, VRL-2 protein may be recovered to provide VRL-2 in active form, capable□of binding VRL-2-specific ligands. Several VRL-2 purification procedures are available and suitable for use.

Recombinant VRL-2 may be purified from cell lysates and extracts, or from conditioned culture medium, by various combinations of, or individual application of salt fractionation, ion exchange chromatography, size exclusion chromatography, hydroxylapatite adsorption chromatography and hydrophobic interaction chromatography. □

Thus, the present invention also provides a process for producing the VRL-2 receptor polypeptides, which comprises culturing a host cell comprising the expression vector containing the above DNA molecule under conditions sufficient for the production of said polypeptide and recovering the polypeptide from the culture medium. The conditions for producing the polypeptides can be easily designed by a skilled person. Well known techniques for refolding proteins may be employed to regenerate active conformation when the polypeptide is denatured during intracellular synthesis, isolation and or purification.

### Antibodies

The present invention further provides antibodies immunospecific for the VRL-2 receptor polypeptide.

Th antibodies may be obtained by administering the polypeptides or epitope-bearing fragments, analogs or cells to an animal, preferably a non-human animal, according to known techniques. For preparation of monoclonal antibodies, any technique which provides antibodies produced by continuous cell line cultures can be used. Examples include the hybridoma technique (Kohler, G. and Milstein, C., Nature (1975) 256:495-497), the trioma technique, the human B-cell hybridoma technique (Kozboret al., Immunology Today (1983) 4:72) and the EBV-hybridoma technique (Cole et al., Monoclonal Antibodies and Cancer Therapy 77-96, Alan R. Liss, Inc., 1985). Techniques for the production of single chain antibodies, such as those described in U.S. Patent No. 4,946,778, can also be adapted to produce single chain antibodies to polypeptides of this invention. Also, transgenic mice, or other organisms, including other mammals, may be used to express humanized antibodies. The above-described antibodies may be employed to isolate or to identify clones expressing the polypeptide or to purify the polypeptides by affinity chromatography. Antibodies against polypeptides of the present invention may also be employed to treat the diseases, amongst others.

### Diagnostic Tool

The DNA molecules of the present invention can be used as diagnostic reagents. Detection of a mutated form of the gene characterized by the polynucleotide of this invention, which is associated with a dysfunction, will provide a diagnostic tool that can add to, or define, a diagnosis of a disease, or susceptibility to a disease, which results from under-expression, over-expression or altered spatial or temporal expression of the gene. Individuals carrying mutations in the gene may be detected at the DNA level by a variety of techniques.

Nucleic acids for diagnosis may be obtained from a subject's cells, such as from blood, urine, saliva tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled VRL-2 nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing (see, e.g., Myers et al.. Science (1985) 230: 1242).

Sequence changes at specific locations may also be revealed by nuclease protection assays, such as RNase and S I protection or the chemical cleavage method (see Cottonet al., Proc Nati Acad Sci USA (1985) 85: 4397-440 1). In another embodiment, an array of oligonucleotides probes comprising VRL-2 nucleotide sequence or fragments thereof can be constructed to conduct efficient screening of e,g., genetic mutations. Array technology methods are well known and have general applicability and can be used to address a variety of questions in molecular genetics including gene expression, genetic linkage, and genetic variability (see for example: M.Chee et al.. Science, Vol 274, pp 610-613 (1996)).

The diagnostic assays offer a process for diagnosing or determining a susceptibility to the VRL-2 related diseases through detection of mutation in the VRL-2 gene by the methods described. In addition, such diseases may be diagnosed by methods comprising determining from a sample derived from a subject an abnormally decreased or increased level of polypeptide or mRNA. Decreased or increased expression can be measured at the RNA level using any of the methods well known in the art for the quantitation of polynucleotides, such as, for example, nucleic acid amplification, for instance PCR, RT-PCR. RNase protection, Northern plotting and other hybridization methods. Assay techniques that can be used to determine levels of a protein, such as a polypeptide of the present invention, in a sample derived from a host are well-known to those of skill in the art. Such assay methods include radioimmunoassays, competitive-binding assays, Western Blot analysis and ELISA assays.

In another aspect, the present invention relates to a diagnostic kit which comprises the DNA molecule of the present invention or an antibody to the polypeptide of the present invention. If desired, the diagnostic kit can comprise other materials which can be used in known diagnostic kits. Such a kit can be used in diagnosing a disease or suspectability to a disease, particularly pain, chronic pain, neuropathic pain, postoperative pain, rheumatoid arthritic pain, neuralgia, neuropathies, algesia, nerve injury, ischaemia, neurodegeneration, stroke, incontinence and inflammatory disorders, amongst others.

### Screening Methods

The novel VRL-2 receptor of the present invention is suitable for use in an assay procedure for identification of modulators which modulate the function of the VRL-2 receptor activity. Thus, this invention also provides a method for screening modulators which modulate the function of the VRL-2 polypeptides. As used herein, "modulating function of a receptor" or "modulating receptor activity" includes the inhibition or activation of the receptor, and also includes directly or indirectly affecting the normal regulation of the receptor activity. As used herein, the term "modulator" means compounds, agonists, antagonists, ligands, substrates and enzymes which directly or indirectly affect regulation of the receptor activity.

The VRL-2 receptor of the present invention may be obtained from both native and recombinant sources for use in an assay procedure to identify receptor modulators. In general, an assay procedure to identify VRL-2 receptor modulators may comprise contacting the VRL-2 polypeptide (or the cells or membranes bearing the polypeptide) of the present invention with a test compound or sample which expects to contain a VRL-2 receptor modulator, and detecting an activity to modulate the function of the VRL-2 receptor (e.g., binding activity). The test compounds or samples may be tested directly on, for example, purified receptor, subcellular fractions of receptor-producing cells, and/or whole cells expressing the receptor. The test compound or sample may be added to the receptor in the presence or absence of a known labelled or unlabelled receptor ligand. Further, these screening methods may test whether the candidate compound results in a signal generated by activation or inhibition of the polypeptide, using detection systems appropriate to the cells bearing the polypeptide. The modulating activity of the test compound or sample may be determined by, for example, analyzing the ability of the test compound or_sample to bind to the receptor, activate the receptor, inhibit receptor activity, inhibit or enhance the binding of other compounds to the receptor, modifying receptor regulation, or modifying an intracellular activity.

The other known screening methods can be used in this invention. Such known methods can be found in PCT International Publication No. WO 99/37765, Page 13, Line 5 to Page 14, Line 31. In addition, the screening method of this invention covers a counter-assay to identify a certain compound which does not have a VRL-2 modulating activity.

The above-mentioned screening method can be practiced by use of a screening kit for identifying modulators for polypeptides of the present invention, which comprises (a) a polypeptide of the present invention; (b) a recombinant cell expressing a polypeptide of the present invention; or (c) a cell membrane expressing a polypeptide of the present invention. Any known materials or compoents which can be used for a screening kit can be added to the screening kit of this invention.

The modulators of VRL-2 receptor activity are useful in treating disease states involving the VRL-2 receptor activity as mentioned above.

### Pharmaceutical Composition

The present invention further provides a pharmaceutical composition for treatment of conditions associated with biological function of a human vanilloid receptor-like protein, which comprises a therapeutically effective amount of a modulator (e.g., agonist or antagonist) obtained by the above-mentioned screening methods and a pharmaceutically acceptable carrier. The conditions associated with biological function of the VRL-2 receptor include pain, nociceptive pain, chronic pain, neuropathic pain, postoperative pain, cancer pain, rheumatoid arthritic pain, osteoarthritis, diabetic neuropathies, neuralgia, neuropathies, algesia, nerve injury, muscle-skeletal pain, low back pain, neurodegeneration, stroke, inflammatory disorders, athma, allergy, urogenital disorders, incontinence, hypertension, hypotension, perivasular disease and the like. Thus, the pharmaceutical compositions of this invention are useful as drugs for treating such diseases.

The pharmaceutical composition of this invention can be administered via either the oral, parenteral or topical routes to mammals. In general, these compounds are most desirably administered to humans in doses ranging from 0.1 mg to 750 mg per day, preferably from 10 mg to 500 mg per day, although variations will necessarily occur depending upon the weight and condition of the subject being treated, the disease state being treated and the particular route of administration chosen.

The compounds of the present invention may be administered alone or in combination with pharmaceutically acceptable carriers or diluents by either of the above routes previously indicated, and such administration can be carried out in single or multiple doses. More particularly, the novel therapeutic agents of the invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, aqueous suspensions, injectable solutions, elixirs, syrups, and the like. Such carriers include solid diluents or fillers, sterile aqueous media and various nontoxic organic solvents, etc. Moreover, oralpharmaceutical compositions can be suitably sweetened and/or flavored. In general, the therapeutically-effective compounds of this invention are present in such dosage forms at concentration levels ranging 5% to 70% by weight, preferably 10% to 50% by weight.

For oral administration, tablets containing various excipients such as microcrystalline cellulose, sodium citrate, calcium carbonate, dipotassium phosphate and glycine may be employed along with various disintegrants such as starch and preferably corn, potato or tapioca starch, alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatine capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a compound of the present invention in either sesame or peanut oil or in aqueous propylene glycol may be employed. The aqueous solutions should be suitably buffered (preferably pH>8) if necessary and the liquid diluent first rendered isotonic. These aqueous solutions are suitable for intravenous injection purposes. The oily solutions are suitable for intra-articular, intra-muscular and subcutaneous injection purposes. The preparation of all these solutions under sterile conditions is readily accomplished by standard pharmaceutical techniques well-known to those skilled in the art. Additionally, it is also possible to administer the compounds of the present invention topically when treating inflammatory conditions of the skin and this may preferably be done by way of creams, jellies, gels, pastes, ointments and the like, in accordance with standard pharmaceutical practice.

### Transgenic Animals

The polypeptides and/or DNA molecules of the present invention can be used to generate genetically modified non-human animals or site specific gene modifications in cell lines. The term "transgenic" is intended to encompass genetically modified animals having, for example, a deletion or other knock-out of VRL-2 receptor gene activity, an exogenous VRL-2 receptor gene that is stably transmitted in the host cells, a "knock-in" having altered VRL-2 receptor gene expression, or an exogenous VRL-2 receptor polypeptide promoter operably linked to a reporter gene. Homozygous and heterozygous knock-outs and knock-ins of VRL-2 receptor polypeptide function are particularly preferred.

Transgenic animals can be made according to methods known to skilled persons in the art. For example, the transgenic animals may be made through homologous recombination, where the endogenous VRL-2 receptor locus is altered. Alternatively, a nucleic acid construct is randomly integrated into the genome. Vectors for stable integration include plasmids, retroviruses and other animal viruses, YACs, and the like. Of interest are transgenic mammals, preferably a mammal from a genus selected from the group consisting of Mus (e.g., mice), Rattus (e.g., rats), Oryctologus (e.g., rabbits) and Mesocricetus (e.g., hamsters). More specific descriptions about preparation and use of the transgenic animals can be found in WO 99/37675, Page 34, Line 36 to Page 37, Line 12.

### EXAMPLES

The following examples are provided for the purpose of illustrating the present invention without, however, limiting the same thereto.

### Example 1: Cloning of h VRL-2 cDNA

A BLAST search against LifeSeq ™ database (Incyte) was made, using the polynucleotide and polypeptide sequences of the rat vanilloid receptor subtype 1 (hereinafter referred to as "VR1"). Six cDNA clones were obtained that contained nucleotide sequences highly homologous to the rat VR1. Five clones out of six were human homologs of rat VR1 or human vanilloid receptor-like protein 1 (hereinafter referred to as "VRL-1"). However, one cDNA clone did not match either of VR1 or VRL-1, and encoded a novel human receptor homologous to vanilloid receptor subtypes. This cDNA was designated as "VRL-2". This cDNA clone did not contain the entire open reading frame.

Further, a full-length cDNA cloning of human VRL-2 was performed. To identify start codon of VRL-2, two primers (5'-GCT GTC GGG GAA GAG GCG GGC ACA CTT G-3' and 5'-GCA GCA GTT CAT TGA TGG GCT CCA CAG C) designed from the sequence data of Incyte clone and human pancreas Marathon-Ready™ cDNA (Clontech, Palo Alto, Calif.) were used. 5' rapid amplification of cDNA ends (5'RACE) was performed under the following conditions: 94°C for 1 min and 68°C for 2 min, 30 cycles. The amplified 1.1 Kb RACE product were cloned and sequenced by automated DNA sequencer ABI PRISM 310 (PE Biosystems, Foster City, Calif.) produced by TOYOBO. These sequence data showed that 5'RACE products contained two kinds of 5' portion that possess a potential initiator codon downstream of an in-frame termination codon. VRL-2 constituted a subfamily that contained at least two spliced variants. One clone was designated as VRL-2a, and the other was designated as VRL-2b.

To isolate complete open reading flame of VRL2a and VRL-2b, PCR was performed using Advantage-HF PCR Kit (Clontech, Palo Alto, Calif.) and human kidney cDNA library (Clontech, Palo Alto, Calif.). Two sets of primers were designed from sequence data of 5'RACE products and Incyte clone; VRL-2a Forward 5'-ATC TGC GCA TGA AGTTCC AG-3', VRL-2b Forward 5'-GAC ATC GCG GAG CGC ACC GGC AAC ATG-3' and VRL-2a,b Reverse 5'-GCT GGA CTA GAA ATG AGT GGG CAG AGA A. PCR was performed under the following conditions: 94°C for 20 sec, 58°C for 30 sec and 72°C for 30 sec, 25 cycles. PCR products of 2.6 kb (VRL-2a) and 1.9 kb (VRL-2b) were cloned and sequenced by automated DNA sequencer ABI PRISM 310 (PE Biosystems, Foster City, Calif.; produced by TOYOBO Gene Analysis. The amino acid sequences of VRL-2a and VRL-2b are shown in SEQ ID NO:2 and SEQ ID NO:4, respectively.

### Example 2: Expression of hVRL-2 in Mammalian Cells

A cDNA encoding human VRL2a was subcloned into the eukaryotic expression vector pcDNA3.1 (Invitrogen). CHO-K1 cells and HEK 293 cells were plated onto poly-1-lysine coated glass-bottom dishes and transiently transfected with expression vector and pIRES2-EGFP (Clonetech) (total 1.0 µg) and 3 µl FuGENE6 transfection reagent (Roche) in 97 µl of OptiMEM medium (Life Technologies) per 35mm dish.

### Example 3: Functional Analysis of hVRL-2

Activities of VRL-2 as a cation channel are readily detected with fluorescent dyes and conventional microscopy. The VRL-2-expressing cells (as obtained in Example 2) plated on glass coverslips are loaded with calcium indicators such as fura-2 or fluo-3, or a membrane potential indicator DiBAC4 in Hank's balanced salt buffer. The osmotic, mechanical or thermal stimuli are applied to the VRL-2-expressing cells loaded with the fluorescent dyes, and changes in fluorescence are monitored by a single cell imaging system mounted on an inverted fluorescence microscopy.

In this example, Ca²⁺ measurements were carried out for 24 to 48 hours after transfection. Ca²⁺ measurements in single cells were made using the fluorescent Ca²⁺ indicator fura-2 in combination with a fluorescence imaging system MERLIN (OLYMPUS) mounted on an inverted fluorescence microscopy (IX70, OLYMPUS). For Ca²⁺ imaging, cells were loaded with 5 µM fura-2 acetoxymethylester (Dojin) for 30 min at room temperature in isotonic (300 mosmol 1⁻¹) measuring buffer containing 88 mM NaCl, 5 mM KCl, 1 mM MgCl₂, 1 mM CaCl₂, 5.5 mM glucose and 10 mM HEPES at pH7.4. The osmolarity was adjusted by addition of appropriate amounts of mannitol. A sequence of fluorescent images (340 nm/380 nm excitation) was taken to monitor Ca²⁺ influx response in cells with or without expression of GFP before and after the osmotic change. Cells were initially kept in isotonic solution. Hypotonic challenge was achieved by perfusion with 200 mosmol l⁻¹ measuring buffer. The solution of lower osmolarity was obtained by reducing the mannitol concentration. The effective assay buffer volume was 2 ml, and perfusion was carried out at room temperature at a flow rate of 3 ml min⁻¹. Complete exchange of the perfusion solution was assumed after 2 min. Non-transfected cells were used as controls. Other control experiments were carried out using cells transfected with pcDNA3.1.

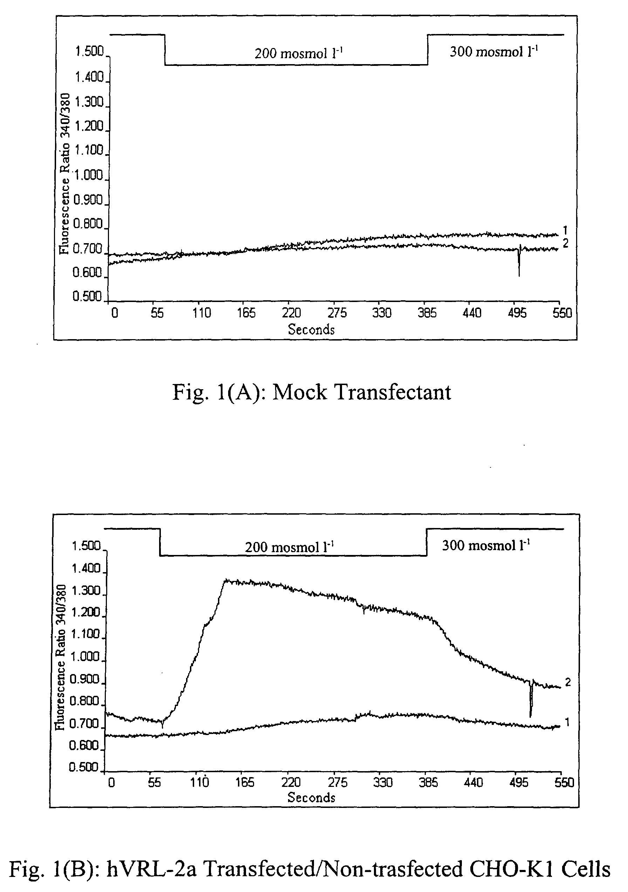

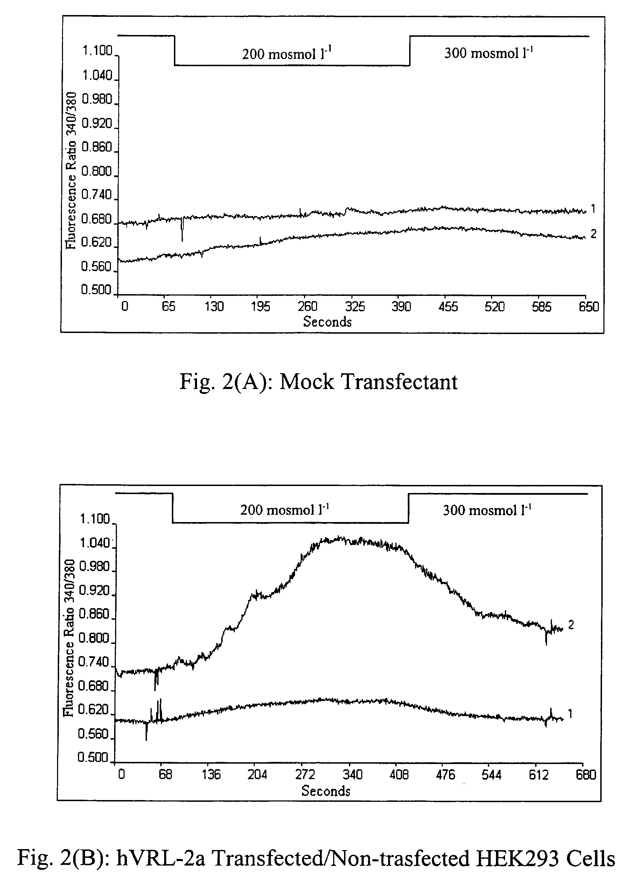

The results of the experiments can be seen in Figs 1(A), 1(B), 2(A) and 2(B).

Figs.1(A) and 1(B) show Ca²⁺ increase in hVRL-2 transfected CHO-K1 cells in response to a reduction in extracellular osmolarity. In Fig. 1(A), Lines 1 and 2 show response curves of cells without expression vectors (Mock transfection). In Fig. 1(B), Line 1 shows a response curve of a GFP-negative cell (non-transfected cell), and Line 2 shows a response curve of a GFP-positive cell (hVRL-2a transfected cell).

Figs.2(A) and 2(B) show Ca²⁺ increase in hVRL-2 transfected HEK293 cells in response to a reduction in extracellular osmolarity. In Fig. 2(A), Lines 1 and 2 show response curves of cells without expression vectors (Mock transfection). In Fig. 2(B), Line 1 shows a response curve of a GFP-negative cell (non-transfected cell), and Line 2 shows a response curve of a GFP-positive cell (hVRL-2a transfected cell).

As can be seen in these Figures, upon reduction of extracellular osmolarity, intracellular Ca²⁺ increased significantly in the hVRL2a transfected cells, but not in control cells. This response was reversible, and increasing the osmolarity led to a decrease in intracellular Ca²⁺.

All publications, including but not limited to patents and patent applications, cited in this specification are herein incorporated by reference as if each individual publication were specifically and individually indicated to be incorporated by reference herein as though fully set forth. In particular, the definition of technical terms described in WO 99/37765 can be used as reference.

### Annex to the application documents - subsequently filed sequences listing

<210> 5
<211> 28
<212> DNA
<213> Homo sapiens

<400> 5
gctgtcgggg aagaggcggg cacacttg


<210> 6
<211> 28
<212> DNA
<213> Homo sapiens

<400> 6
gcagcagttc attgatgggc tccacagc


<210> 7
<211> 20
<212> DNA
<213> Homo sapiens

<400> 7
atctgcgcat gaagttccag


<210> 8
<211> 27
<212> DNA
<213> Homo sapiens

<400> 8
gacatcgcgg agcgcaccgg caacatg


<210> 9
<211> 28
<212> DNA
<213> Homo sapiens

<400> 9
gctggactag aaatgagtgg gcagagaa

SEQUENCE LISTING (which forms part of the description)

<110> Pfizer, Inc.

<120> Human Vanilloid Receptor-Like Proteins

<130> PC9979A

<140>
<141>

<150> US 60/208,156
<151> 2000-05-31

<160> 4

<170> PatentIn Ver. 2.1

<210> 1
<211> 2749
<212> DNA
<213> Human

<220>
<221> CDS
<222> (85)..(2700)

<400> 1
aaccgcttga ggatttaagc tttgccactt tggctccgga gcaagggcag agggctgagc 60
agtgcagacg ggcctggggc aggc atg gcg gat tcc agc gaa ggc ccc cgc 111
gcg ggg ccc ggg gag gtg gct gag ctc ccc ggg gat gag agt ggc acc 159
cca ggt ggg gag gct ttt cct ctc tcc tcc ctg gcc aat ctg ttt gag 207
ggg gag gat ggc tcc ctt tcg ccc tca ccg gct gat gcc agt cgc cct 255
gct ggc cca ggc gat ggg cga cca aat ctg cgc atg aag ttc cag ggc 303
gcc ttc cgc aag ggg gtg ccc aac ccc atc gat ctg ctg gag tcc acc 351
cta tat gag tcc tcg gtg gtg cct ggg ccc aag aaa gca ccc atg gac 399
tca ctg ttt gac tac ggc acc tat cgt cac cac tcc agt gac aac aag 447
agg tgg agg aag aag atc ata gag aag cag ccg cag agc ccc aaa gcc     495
cct gcc cct cag ccg ccc ccc atc ctc aaa gtc ttc aac cgg cct atc     543
ctc ttt gac atc gtg tcc cgg ggc tcc act gct gac ctg gac ggg ctg     591
ctc cca ttc ttg ctg acc cac aag aaa cgc cta act gat gag gag ttt     639
cga gag cca tct acg ggg aag acc tgc ctg ccc aag gcc ttg ctg aac     687
ctg agc aat ggc cgc aac gac acc atc cct gtg ctg ctg gac atc gcg     735
gag cgc acc ggc aac atg cgg gag ttc att aac tcg ccc ttc cgt gac     783
atc tac tat cga ggt cag aca gcc ctg cac atc gcc att gag cgt cgc     831
tgc aaa cac tac gtg gaa ctt ctc gtg gcc cag gga gct gat gtc cac     879
gcc cag gcc cgt ggg cgc ttc ttc cag ccc aag gat gag ggg ggc tac     927
ttc tac ttt ggg gag ctg ccc ctg tcg ctg gct gcc tgc acc aac cag     975
ccc cac att gtc aac tac cta acg gag aac ccc cac aag aag gcg gac     1023
atg cgg cgc cag gac tcg cga ggc aac aca gtg ctg cat gcg ctg gtg     1071
gcc att gct gac aac acc cgt gag aac acc aag ttt gtt acc aag atg     1119
tac gac ctg ctg ctg ctc aag tgt gcc cgc ctc ttc ccc gac agc aac     1167
ctg gag gcc gtg ctc aac aac gac ggc ctc tcg ccc ctc atg atg gct     1215
gcc aag acg ggc aag att ggg atc ttt cag cac atc atc cgg cgg gag     1263
gtg acg gat gag gac aca cgg cac ctg tcc cgc aag ttc aag gac tgg     1311
gcc tat ggg cca gtg tat tcc tcg ctt tat gac ctc tcc tcc ctg gac     1359
acg tgt ggg gaa gag gcc tcc gtg ctg gag atc ctg gtg tac aac agc     1407
aag att gag aac cgc cac gag atg ctg gct gtg gag ccc atc aat gaa     1455
ctg ctg cgg gac aag tgg cgc aag ttc ggg gcc gtc tcc ttc tac atc     1503
aac gtg gtc tcc tac ctg tgt gcc atg gtc atc ttc act ctc acc gcc     1551
tac tac cag ccg ctg gag ggc aca ccg ccg tac cct tac cgc acc acg     1599
gtg gac tac ctg cgg ctg gct ggc gag gtc att acg ctc ttc act ggg     1647
gtc ctg ttc ttc ttc acc aac atc aaa gac ttg ttc atg aag aaa tgc     1695
cct gga gtg aat tct ctc ttc att gat ggc tcc ttc cag ctg ctc tac     1743
ttc atc tac tct gtc ctg gtg atc gtc tca gca gcc ctc tac ctg gca     1791
ggg atc gag gcc tac ctg gcc gtg atg gtc ttt gcc ctg gtc ctg ggc     1839
tgg atg aat gcc ctt tac ttc acc cgt ggg ctg aag ctg acg ggg acc     1887
tat agc atc atg atc cag aag att ctc ttc aag gac ctt ttc cga ttc     1935
ctg ctc gtc tac ttg ctc ttc atg atc ggc tac gct tca gcc ctg gtc     1983
tcc ctc ctg aac ccg tgt gcc aac atg aag gtg tgc aat gag gac cag 2031
acc aac tgc aca gtg ccc act tac ccc tcg tgc cgt gac agc gag acc 2079
ttc agc acc ttc ctc ctg gac ctg ttt aag ctg acc att ggc atg ggc 2127
gac ctg gag atg ctg agc agc acc aag tac ccc gtg gtc ttc atc atc 2175
ctg ctg gtg acc tac atc atc ctc acc ttt gtg ctg ctc ctc aac atg 2223
ctc att gcc ctc atg ggc gag aca gtg ggc cag gtc tcc aag gag agc 2271
aag cac atc tgg aag ctg cag tgg gcc acc acc atc ctg gac att gag 2319
cgc tcc ttc ccc gta ttc ctg agg aag gcc ttc cgc tct ggg gag atg 2367
gtc acc gtg ggc aag agc tcg gac ggc act cct gac cgc agg tgg tgc 2415
ttc agg gtg gat gag gtg aac tgg tct cac tgg aac cag aac ttg ggc 2463
atc atc aac gag gac ccg ggc aag aat gag acc tac cag tat tat ggc 2511
ttc tcg cat acc gtg ggc cgc ctc cgc agg gat cgc tgg tcc tcg gtg 2559
gta ccc cgc gtg gtg gaa ctg aac aag aac tcg aac ccg gac gag gtg 2607
gtg gtg cct ctg gac agc atg ggg aac ccc cgc tgc gat ggc cac cag 2655
cag ggt tac ccc cgc aag tgg agg act gat gac gcc ccg ctc tag 2700
ggactgcagc ccagccccag cttctctgcc cactcatttc tagtccagc          2749

<210> 2
<211> 871
<212> PRT
<213> Human

<400> 2

<210> 3
<211> 1900
<212> DNA
<213> Human

<220>
<221> CDS
<222> (43)..(1851)

<400> 3
gcggccgctg aattctagga catcgcggag cgcaccggca ac atg cgg gag ttc     54
                                               Met Arg Glu Phe
                                               1

att aac tcg ccc ttc cgt gac atc tac tat cga ggg gag ctg ccc ctg     102
Ile Asn Ser Pro Phe Arg Asp Ile Tyr Tyr Arg Gly Glu Leu Pro Leu
5                   10                  15                  20

tcg ctg gct gcc tgc acc aac cag ccc cac att gtc aac tac ctg acg     150
Ser Leu Ala Ala Cys Thr Asn Gln Pro His Ile Val Asn Tyr Leu Thr
                25                  30                  35

gag aac ccc cac aag aag gcg gac atg cgg cgc cag gac tcg cga ggc     198
Glu Asn Pro His Lys Lys Ala Asp Met Arg Arg Gln Asp Ser Arg Gly
            40                  45                  50

aac aca gtg ctg cat gcg ctg gtg gcc att gct gac aac acc cgt gag     246
Asn Thr Val Leu His Ala Leu Val Ala Ile Ala Asp Asn Thr Arg Glu
        55                  60                  65

aac acc aag ttt gtt acc aag atg tac gac ctg ctg ctg ctc aag tgt     294
Asn Thr Lys Phe Val Thr Lys Met Tyr Asp Leu Leu Leu Leu Lys Cys
    70                  75                  80

gcc cgc ctc ttc ccc gac agc aac ctg gag gcc gtg ctc aac aac gac     342
Ala Arg Leu Phe Pro Asp Ser Asn Leu Glu Ala Val Leu Asn Asn Asp
85                  90                  95                  100

ggc ctc tcg ccc ctc atg atg gct gcc aag acg ggc aag att ggg atc     390
Gly Leu Ser Pro Leu Met Met Ala Ala Lys Thr Gly Lys Ile Gly Ile
                105                 110                 115

ttt cag cac atc atc cgg cgg gag gtg acg gat gag gac aca cgg cac     438
Phe Gln His Ile Ile Arg Arg Glu Val Thr Asp Glu Asp Thr Arg His
            120                 125                 130

ctg tcc cgc aag ttc aag gac tgg gcc tat ggg cca gtg tat tcc tcg     486
Leu Ser Arg Lys Phe Lys Asp Trp Ala Tyr Gly Pro Val Tyr Ser Ser
        135                 140                 145

ctt tat gac ctc tcc tcc ctg gac acg tgt ggg gaa gag gcc tcc gtg     534
Leu Tyr Asp Leu Ser Ser Leu Asp Thr Cys Gly Glu Glu Ala Ser Val
    150                 155                 160

ctg gag atc ctg gtg tac aac agc aag att gag aac cgc cac gag atg     582
Leu Glu Ile Leu Val Tyr Asn Ser Lys Ile Glu Asn Arg His Glu Met
165                 170                 175                 180

ctg gct gtg gag ccc atc aat gaa ctg ctg cgg gac aag tgg cgc aag     630
Leu Ala Val Glu Pro Ile Asn Glu Leu Leu Arg Asp Lys Trp Arg Lys
                185                 190                 195

ttc ggg gcc gtc tcc ttc tac atc aac gtg gtc tcc tac ctg tgt gcc     678
Phe Gly Ala Val Ser Phe Tyr Ile Asn Val Val Ser Tyr Leu Cys Ala
            200                 205                 210

atg gtc atc ttc act ctc acc gcc tac tac cag ccg ctg gag ggc aca     726

Met Val Ile Phe Thr Leu Thr Ala Tyr Tyr Gln Pro Leu Glu Gly Thr
        215                 220                 225

ccg ccg tac cct tac cgc acc acg gtg gac tac ctg cgg ctg gct ggc     774
Pro Pro Tyr Pro Tyr Arg Thr Thr Val Asp Tyr Leu Arg Leu Ala Gly
    230                 235                 240

gag gtc att acg ctc ttc act ggg gtc ctg ttc ttc ttc acc aac atc     822
Glu Val Ile Thr Leu Phe Thr Gly Val Leu Phe Phe Phe Thr Asn Ile
245                 250                 255                 260

aaa gac ttg ttc atg aag aaa tgc cct gga gtg aat tct ctc ttc att     870
Lys Asp Leu Phe Met Lys Lys Cys Pro Gly Val Asn Ser Leu Phe Ile
                265                 270                 275

gat ggc tcc ttc cag ctg ctc tac ttc atc tac tct gtc ctg gtg atc     918
Asp Gly Ser Phe Gln Leu Leu Tyr Phe Ile Tyr Ser Val Leu Val Ile
            280                 285                 290

gtc tca gca gcc ctc tac ctg gca ggg atc gag gcc tac ctg gcc gtg     966
Val Ser Ala Ala Leu Tyr Leu Ala Gly Ile Glu Ala Tyr Leu Ala Val
        295                 300                 305

atg gtc ttt gcc ctg gtc ctg ggc tgg atg aat gcc ctt tac ttc acc     1014
Met Val Phe Ala Leu Val Leu Gly Trp Met Asn Ala Leu Tyr Phe Thr
    310                 315                 320

cgt ggg ctg aag ctg acg ggg acc tat agc atc atg atc cag aag att     1062
Arg Gly Leu Lys Leu Thr Gly Thr Tyr Ser Ile Met Ile Gln Lys Ile
325                 330                 335                 340

ctc ttc aag gac ctt ttc cga ttc ctg ctc gtc tac ttg ctc ttc atg     1110
Leu Phe Lys Asp Leu Phe Arg Phe Leu Leu Val Tyr Leu Leu Phe Met
                345                 350                 355

atc ggc tac gct tca gcc ctg gtc tcc ctc ctg aac ccg tgt gcc aac     1158
Ile Gly Tyr Ala Ser Ala Leu Val Ser Leu Leu Asn Pro Cys Ala Asn
            360                 365                 370

atg aag gtg tgc aat gag gac cag acc aac tgc aca gtg ccc act tac     1206
Met Lys Val Cys Asn Glu Asp Gln Thr Asn Cys Thr Val Pro Thr Tyr
        375                 380                 385

ccc tcg tgc cgt gac agc gag acc ttc agc acc ttc ctc ctg gac ctg     1254
Pro Ser Cys Arg Asp Ser Glu Thr Phe Ser Thr Phe Leu Leu Asp Leu
    390                 395                 400

ttt aag ctg acc atc ggc atg ggc gac ctg gag atg ctg agc agc acc     1302
Phe Lys Leu Thr Ile Gly Met Gly Asp Leu Glu Met Leu Ser Ser Thr
405                 410                 415                 420

aag tac ccc gtg gtc ttc atc atc ctg ctg gtg acc tac atc atc ctc     1350
Lys Tyr Pro Val Val Phe Ile Ile Leu Leu Val Thr Tyr Ile Ile Leu
                425                 430                 435

acc ttt gtg ctg ctc ctc aac atg ctc att gcc ctc atg ggc gag aca     1398
Thr Phe Val Leu Leu Leu Asn Met Leu Ile Ala Leu Met Gly Glu Thr
            440                 445                 450

gtg ggc cag gtc tcc aag gag agc aag cac atc tgg aag ctg cag tgg     1446
Val Gly Gln Val Ser Lys Glu Ser Lys His Ile Trp Lys Leu Gln Trp
        455                 460                 465

gcc acc acc atc ctg gac att gag cgc tcc ttc ccc gta ttc ctg agg     1494
Ala Thr Thr Ile Leu Asp Ile Glu Arg Ser Phe Pro Val Phe Leu Arg
    470                 475                 480

aag gcc ttc cgc tct ggg gag atg gtc acc gtg ggc aag agc tcg gac     1542
Lys Ala Phe Arg Ser Gly Glu Met Val Thr Val Gly Lys Ser Ser Asp
485                 490                 495                 500

ggc act cct gac cgc agg tgg tgc ttc agg gtg gat gag gtg aac tgg     1590
Gly Thr Pro Asp Arg Arg Trp Cys Phe Arg Val Asp Glu Val Asn Trp
                505                 510                 515

tct cac tgg aac cag aac ttg ggc atc atc aac gag gac ccg ggc aag     1638
Ser His Trp Asn Gln Asn Leu Gly Ile Ile Asn Glu Asp Pro Gly Lys
            520                 525                 530

aat gag acc tac cag tat tat ggc ttc tcg cat acc gtg ggc cgc ctc     1686
Asn Glu Thr Tyr Gln Tyr Tyr Gly Phe Ser His Thr Val Gly Arg Leu
        535                 540                 545

cgc agg gat cgc tgg tcc tcg gtg gta ccc cgc gtg gtg gaa ctg aac     1734
Arg Arg Asp Arg Trp Ser Ser Val Val Pro Arg Val Val Glu Leu Asn
    550                 555                 560

aag aac tcg aac ccg gac gag gtg gtg gtg cct ctg gac agc atg ggg     1782
Lys Asn Ser Asn Pro Asp Glu Val Val Val Pro Leu Asp Ser Met Gly
565                 570                 575                 580

aac ccc cgc tgc gat ggc cac cag cag ggt tac ccc cgc aag tgg agg     1830
Asn Pro Arg Cys Asp Gly His Gln Gln Gly Tyr Pro Arg Lys Trp Arg
                585                 590                 595

act gat gac gcc ccg ctc tag ggactgcagc ccagccccag cttctctgcc     1881
Thr Asp Asp Ala Pro Leu
            600

cactcatttc tagtccagc                                                1900

<210> 4
<211> 602
<212> PRT
<213> Human

<400> 4
Met Arg Glu Phe Ile Asn Ser Pro Phe Arg Asp Ile Tyr Tyr Arg Gly
 1               5                  10                  15
Glu Leu Pro Leu Ser Leu Ala Ala Cys Thr Asn Gln Pro His Ile Val
            20                  25                  30
Asn Tyr Leu Thr Glu Asn Pro His Lys Lys Ala Asp Met Arg Arg Gln
        35                  40                  45
Asp Ser Arg Gly Asn Thr Val Leu His Ala Leu Val Ala Ile Ala Asp
    50                  55                  60
Asn Thr Arg Glu Asn Thr Lys Phe Val Thr Lys Met Tyr Asp Leu Leu
65                  70                  75                  80
Leu Leu Lys Cys Ala Arg Leu Phe Pro Asp Ser Asn Leu Glu Ala Val
                85                  90                  95
Leu Asn Asn Asp Gly Leu Ser Pro Leu Met Met Ala Ala Lys Thr Gly
            100                 105                 110
Lys Ile Gly Ile Phe Gln His Ile Ile Arg Arg Glu Val Thr Asp Glu
        115                 120                 125
Asp Thr Arg His Leu Ser Arg Lys Phe Lys Asp Trp Ala Tyr Gly Pro
    130                 135                 140

Val Tyr Ser Ser Leu Tyr Asp Leu Ser Ser Leu Asp Thr Cys Gly Glu
145                 150                 155                 160
Glu Ala Ser Val Leu Glu Ile Leu Val Tyr Asn Ser Lys Ile Glu Asn
                165                 170                 175
Arg His Glu Met Leu Ala Val Glu Pro Ile Asn Glu Leu Leu Arg Asp
            180                 185                 190
Lys Trp Arg Lys Phe Gly Ala Val Ser Phe Tyr Ile Asn Val Val Ser
        195                 200                 205
Tyr Leu Cys Ala Met Val Ile Phe Thr Leu Thr Ala Tyr Tyr Gln Pro
    210                 215                 220
Leu Glu Gly Thr Pro Pro Tyr Pro Tyr Arg Thr Thr Val Asp Tyr Leu
225                 230                 235                 240
Arg Leu Ala Gly Glu Val Ile Thr Leu Phe Thr Gly Val Leu Phe Phe
                245                 250                 255
Phe Thr Asn Ile Lys Asp Leu Phe Met Lys Lys Cys Pro Gly Val Asn
            260                 265                 270
Ser Leu Phe Ile Asp Gly Ser Phe Gln Leu Leu Tyr Phe Ile Tyr Ser
        275                 280                 285
Val Leu Val Ile Val Ser Ala Ala Leu Tyr Leu Ala Gly Ile Glu Ala
    290                 295                 300
Tyr Leu Ala Val Met Val Phe Ala Leu Val Leu Gly Trp Met Asn Ala
305                 310                 315                 320
Leu Tyr Phe Thr Arg Gly Leu Lys Leu Thr Gly Thr Tyr Ser Ile Met
                325                 330                 335
Ile Gln Lys Ile Leu Phe Lys Asp Leu Phe Arg Phe Leu Leu Val Tyr
            340                 345                 350
Leu Leu Phe Met Ile Gly Tyr Ala Ser Ala Leu Val Ser Leu Leu Asn
        355                 360                 365
Pro Cys Ala Asn Met Lys Val Cys Asn Glu Asp Gln Thr Asn Cys Thr
    370                 375                 380
Val Pro Thr Tyr Pro Ser Cys Arg Asp Ser Glu Thr Phe Ser Thr Phe
385                 390                 395                 400
Leu Leu Asp Leu Phe Lys Leu Thr Ile Gly Met Gly Asp Leu Glu Met
                405                 410                 415
Leu Ser Ser Thr Lys Tyr Pro Val Val Phe Ile Ile Leu Leu Val Thr
            420                 425                 430
Tyr Ile Ile Leu Thr Phe Val Leu Leu Leu Asn Met Leu Ile Ala Leu
        435                 440                 445
Met Gly Glu Thr Val Gly Gln Val Ser Lys Glu Ser Lys His Ile Trp
    450                 455                 460
Lys Leu Gln Trp Ala Thr Thr Ile Leu Asp Ile Glu Arg Ser Phe Pro
465                 470                 475                 480
Val Phe Leu Arg Lys Ala Phe Arg Ser Gly Glu Met Val Thr Val Gly
                485                 490                 495
Lys Ser Ser Asp Gly Thr Pro Asp Arg Arg Trp Cys Phe Arg Val Asp
            500                 505                 510
Glu Val Asn Trp Ser His Trp Asn Gln Asn Leu Gly Ile Ile Asn Glu
        515                 520                 525
Asp Pro Gly Lys Asn Glu Thr Tyr Gln Tyr Tyr Gly Phe Ser His Thr
    530                 535                 540
Val Gly Arg Leu Arg Arg Asp Arg Trp Ser Ser Val Val Pro Arg Val
545                 550                 555                 560
Val Glu Leu Asn Lys Asn Ser Asn Pro Asp Glu Val Val Val Pro Leu
                565                 570                 575
Asp Ser Met Gly Asn Pro Arg Cys Asp Gly His Gln Gln Gly Tyr Pro
            580                 585                 590
Arg Lys Trp Arg Thr Asp Asp Ala Pro Leu
        595                 600

## Claims

1. A polypeptide selected from:
(a) a polypeptide comprising an amino acid sequence of SEQ ID NO:2 or SEQ ID NO:4, or variants thereof; and
(b) a polypeptide having the deduced amino acid sequence translated from the polynucleotide sequence SEQ ID NO:1 or SEQ ID NO:3, or variants thereof.

2. A polypeptide of Claim 1, comprising an amino acid sequence that has at least 75% identity to the an amino acid sequence of SEQ ID NO:2 or SEQ ID NO:4.

3. A polypeptide of Claim, comprising an amino acid sequence that has at least 85% identity to the an amino acid sequence of SEQ ID NO:2 or SEQ ID NO:4.

4. A polypeptide of Claim 1, comprising an amino acid sequence that has at least 95% identity to the an amino acid sequence of SEQ ID NO:2 or SEQ ID NO:4.

5. An isolated and/or purified polynucleotide selected from:
(a) a polynucleotide encoding the polypeptide comprising an amino acid sequence of SEQ ID NO:2 or SEQ ID NO:4;
(b) a polynucleotide having a nucleotide sequence of the 85th to 2700th nucleotides in the nucleotide sequence of SEQ ID NO:1, or a nucleotide sequence of the 43rd to 1851st nucleotides in the nucleotide sequence of SEQ ID NO:3 or variants thereof;
(c) a polynucleotide comprising a nucleotide sequence that has at least 70% identity to the polynucleotide of (a) or (b);
(d) a complement to the polynucleotide of any one of (a) to (c);
(e) a polynucleotide comprising a nucleotide sequence which is capable of hybridising to the polynucleotide of any one of (a) to (d); and
(f) a polynucleotide fragment of the polynucleotide of any one of (a) to (e).

6. A polynucleotide of claim 5, comprising a nucleotide sequence that has at least 75% identity to the nucleotide sequence of the polynucleotide (b).

7. A polynucleotide of claim 5, comprising a nucleotide sequence that has at least 85% identity to the nucleotide sequence of the polynucleotide (b).

8. A polynucleotide of claim 5, comprising a nucleotide sequence that has at least 95% identity to the nucleotide sequence of the polynucleotide (b).

9. A polynucleotide probe or primer comprising at least 15 contiguous nucleotides of the polynucleotide of any one of Claim 5.

10. An expression vector for the expression of a human vanilloid receptor-ike protein in a recombinant host cell wherein said expression vector contains the polynucleotide of Claim 5.

11. A host cell which expresses a human vanilloid receptor-like protein wherein said host cell contains the expression vector of Claim 10.

12. A process for producing a polypeptide of Claim 1, which comprises culturing a host cell of Claim 11 under conditions sufficient for the production of said polypeptide and recovering the polypeptide from the culture medium.

13. An antibody immunospecific for a polypeptide of Claim 1.

14. A diagonostic kit for diagnosing a disease or a susceptibility to a disease associated with biological function of a human vanilloid receptor-like protein, which comprises a polynucleotide of Claim 5, or an antibody to a polypeptide of Claim 1.

15. A method for screening to identify modulators which modulate function of a polypeptide of Claim 1, which comprises contacting a test compound or sample with the polypeptide, and detecting an activity of the test compound or sample to modulate the function of the polypeptide.

16. A modulator obtainable by a screening method of Claim 15.

17. A pharmaceutical composition for treatment of conditions associated with biological function of a human vanilloid receptor-like protein, which comprises a therapeutically effective amount of a modulator of Claim 16 and a pharmaceutically acceptable carrier.

18. The pharmaceutical composition according to claim 17, wherein said conditions are selected from pain, nociceptive pain, chronic pain, neuropathic pain, postoperative pain, cancer pain, rheumatoid arthritic pain, osteoarthritis, diabetic neuropathies, neuralgia, neuropathies, algesia, nerve injury, muscle-skeletal pain, low back pain, neurodegeneration, stroke, inflammatory disorders, asthma, allergy, urogenital disorders, incontinence, hypertension, hypotension and perivascular disease.

19. Use of a modulator according to claim 16 in the manufacture of a medicament for the treatment of a condition associated with biological function of a human vanilloid receptor-like protein.

20. Use according to claim 19, wherein said condition is pain, nociceptive pain, chronic pain, neuropathic pain, postoperative pain, cancer pain, rheumatoid arthritic pain, osteoarthritis, diabetic neuropathies, neuralgia, neuropathies, algesia, nerve injury, muscle-skeletal pain, low back pain, neurodegeneration, stroke, inflammatory disorders, asthma, allergy, urogenital disorders, incontinence, hypertension, hypotension and perivascular disease.

21. A non-human transgenic animal model for vanilloid receptor-like gene function, wherein the transgenic animal has an alteration in vanilloid receptor-like function relative to a normal animal of the same species.
